# EUROPEAN PATENT SPECIFICATION

(11) **EP 0 332 142 B1**
(45) Date of publication and mention of the grant of the patent: **16.08.1995**
(21) Application number: 89104009.9
(22) Date of filing: 07.03.1989
(51) Int. Cl.: C12M 1/40, C12N 11/00

(54) **Method of disinfecting immobilized biocatalysts and reactor provided with fluidized chamber**
Methode zur Entkeimung immobilisierter Biokatalysatoren und mit einem Fliessraum versehener Reaktor
Méthode de désinfection de biocatalyseurs immobilisés et réacteur équipé d'une enceinte fluidisée

(30) Priority: 09.03.1988 JP 55756/88
(43) Date of publication of application: 13.09.1989
(73) Proprietor: SNOW BRAND MILK PRODUCTS CO., LTD., Sapporo-shi, Hokkaido 065 (JP)
(72) Inventor: Shukunobe, Yukitaka, Kawagoe-shi Saitama-ken (JP); Nakamura, Tetsuo, Iruma-shi Saitama-ken (JP); Yoshida, Tomoe, Tokorozawa-shi Saitama-ken (JP); Doki, Ryoichi, Sayama-shi Saitama-ken (JP); Kuwazuru, Mamoru, Kawagoe-shi Saitama-ken (JP); Shimoda, Kouzou, Nerima-ku Tokyo (JP)
(74) Representative: Hoffmeister, Helmut, Dr. Dipl.-Phys.

(56) References cited:
- EP-A- 0 322 749
- DE-A- 3 412 143
- US-A- 3 923 663

## Description

The present invention relates to disinfecting immobilized enzymes or immobilized myceliums (hereinafter referred to as immobilized biocatalyst) by using irradiation of ultraviolet light, and to reducing difficulties with washing and disinfecting immobilized biocatalyst and adsorption. A reactor is used for said washing an disinfecting immobilized biocatalyst, comprising:
- a fluidized chamber with an opening at its lower position,
- a column chamber connected to said fluidized chamber,
- an inner tube extending through the fluidized chamber to the column chamber, said tube being movable vertically inside said fluidized chamber, whereby in the elevated position of the tube the fluidized chamber and the column chamber are connected,
- a lower opening of said inner tube contacting the column chamber in the lower position of the tube, thereby separating the column chamber and the fluidized chamber, but connecting the tube and the column chamber.

The art of production using immobilized biocatalyst has been extremely innovative. There are many reactors such as the packed layer type, the fluidized bed type, the stirred tank type and the membrane type used in this field.

A reactor of the type mentioned is described in EP-A2 0 322 749. In this prior art, during washing and disinfecting of immobilized biocatalyst the inner tube is taken up so as to bring an outer surface of the head opening into contact with an inner surface of an upper surface of funnel portion of the fluidized chamber. Then washing liquid is introduced into the reactor at high pressure through the lower portion of the column chamber connected to the fluidized chamber. By a vortex flow of the washing liquid being generated mainly in the fluidized chamber, the washing and stirring of immobilized biocatalyst and washing of column chamber and apparatus in the fluidized chamber is operated at the same time.

This results in the lowering of activity of immobilized biocatalyst when the washing mainly for the purpose of washing of column chamber and fluidized chamber is operated. Washing mainly for the purpose of washing immobilized biocatalyst does not wash sufficiently column chamber and fluidized chamber.

Further, in the said prior art, disinfection is made by using surface active agent like quaternary ammonium salt after washing immobilized biocatalyst; despite that the effect of disinfection in insufficient.

The present invention provides a reactor for washing and disinfecting immobilized biocatalyst without lowering the activity of immobilized biocatalyst which has effects in disinfection.

The present invention provides a reactor of the type mentioned, which is characterized in that the fluidized chamber is provided with an ultraviolet lamp insertable into the inside of said fluidized chamber.

The present invention has the following advantages:

Using the irradiation of ultraviolet light to immobilize biocatalyst at illuminating power of less than 0.5 mW/cm², the disinfecting effect is considerably more developed than with conventional disinfectants, and therefore lowering of activity of immobilized biocatalyst in prevented. And as for the method of irradiating, both overhead irradiation and dipping irradiation are possible provided that the ceiling value of the irradiation of ultraviolet light is set at illuminating power of less than 0.5 mW/cm².

Further, in the reactor provided with fluidized chamber the washing of immobilized biocatalyst and the washing of a column chamber and apparatus in a fluidized chamber may be operated simultaneously, or separately.

### BRIEF DESCRIPTION OF THE DRAWINGS

The above and other advantages of the invention will be apparent from the reference to the description taken in connection with the accompanying drawings, in which;
- Fig. 1: is a sectional view which shows one embodiment of the reactor provided with fluidized chamber according to the present invention,
- Fig. 2: is a detailed sectional view around the upper cylinder portion 22 in which the outside of the head opening 39 of inner tube 38 is connected with the bottom of the engagement 26 of Fig. 1,
- Fig. 3: through Fig. 6 are graphic charts showing the experimental examples in the method of disinfecting immobilized biocatalyst;

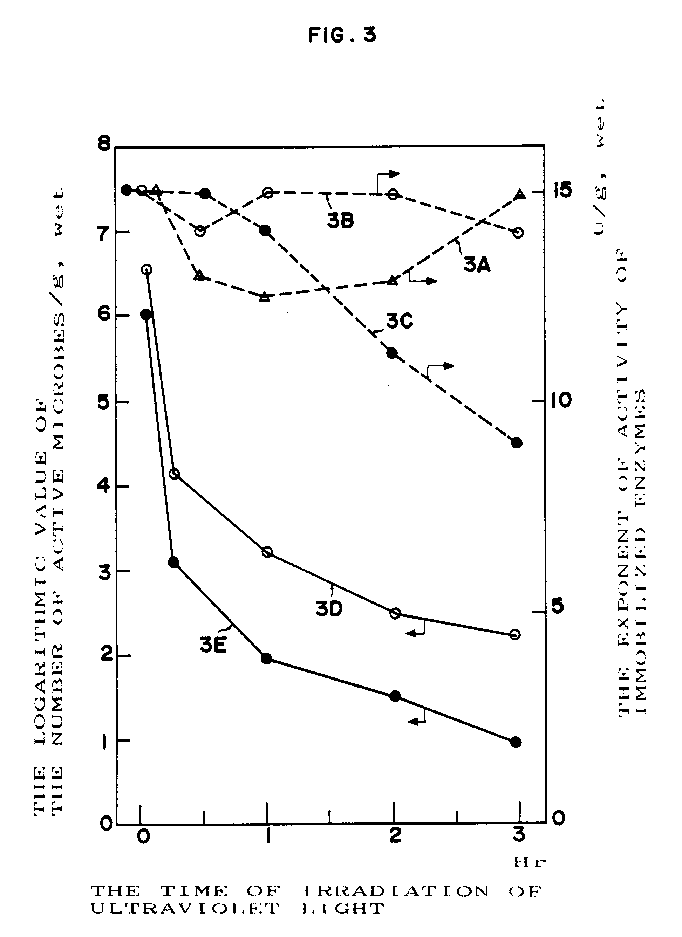

- Fig. 3: is a graphic chart showing effect of disinfection and activity of immobilized biocatalyst as for overhead irradiation and dipping irradiation by using ultraviolet light,

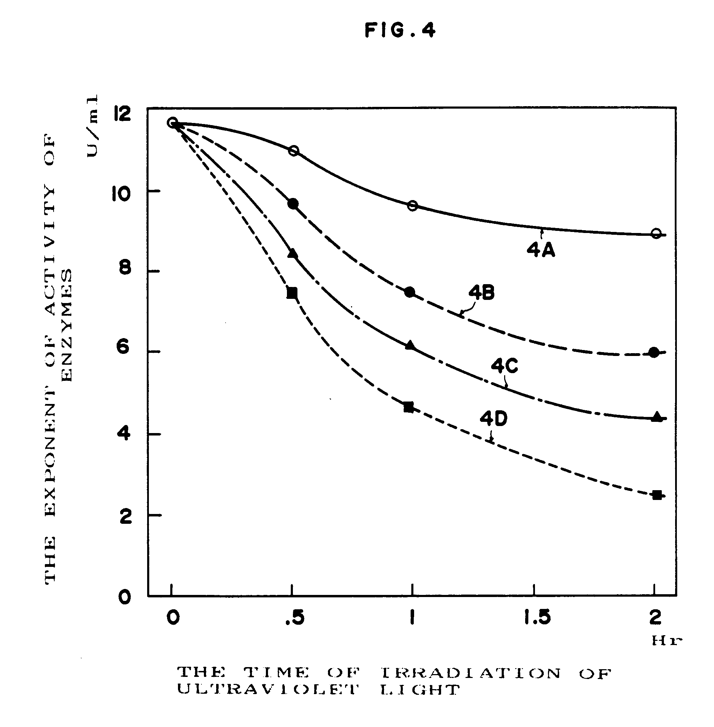

- Fig. 4: is a graphic chart showing the results of irradiating experiment varying the illuminating power of irradiation of ultraviolet light as for the overhead irradiation of Fig. 3, and

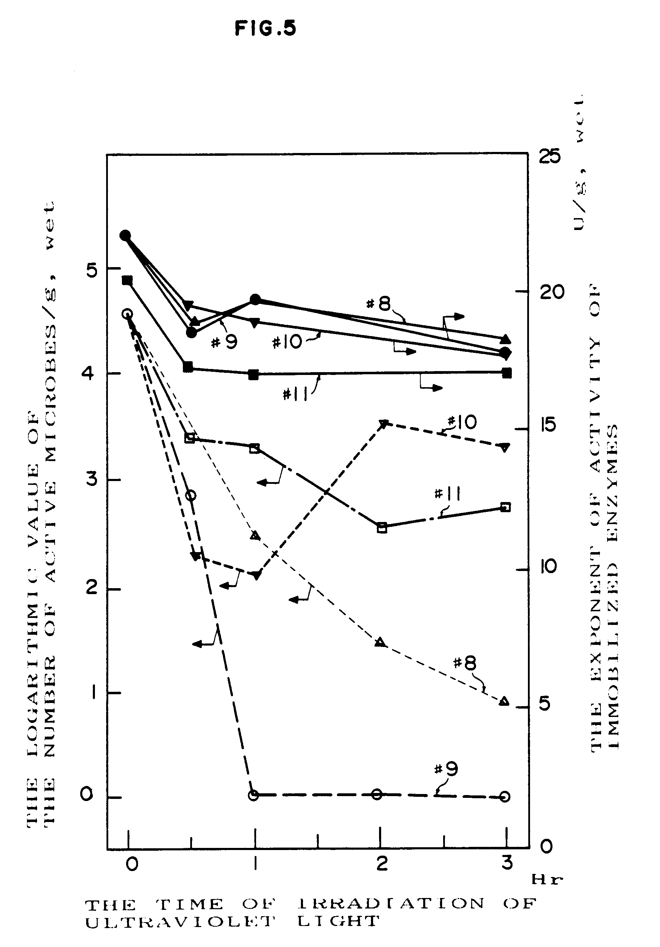

- Fig. 5: is a graphic chart showing the decreasing of the number of active microbes by disinfection using overhead irradiation and dipping irradiation of ultraviolet light at the illuminating power of 0.5 mW/cm².

### DESCRIPTION OF THE INVENTION

In regard to the reactor for disinfection by using ultraviolet light examples are explained as follows:

### EXAMPLE 1

There is made a comparison between the method of irradiating ultraviolet light from above on immobilized biocatalyst solution to the said immobilized biocatalyst solution by using ultraviolet lamp (hereinafter referred to as overhead irradiation) and the method of dipping the ultraviolet lamp into the said immobilized biocatalyst solution (hereinafter referred to as dipping irradiation). In the experiment protease "S" was used as immobilized enzymes and "chitopearl" (trademark) was used as carriers, and illuminating power of irradiation of ultraviolet light was set at 0.5 mW/cm² on the surface of solution by overhead irradiation, and at 4.4 mW/cm² at 1.9 cm from the illuminant by dipping irradiation.

The above experiment resulted in the graphic chart shown as in Fig. 3. Dotted lines 3A, 3B and 3C in Fig. 3 indicate over the time of irradiation of ultraviolet light for the horizontal axis, the exponent of activity of immobilized enzymes for the vertical axis. Solid lines 3D, 3E indicate over the time of irradiation of ultraviolet light for the horizontal axis, the logarithmic value of the number of active microbes for the vertical axis. The following conditions of irradiations of ultraviolet light are used: 3A was made at no irradiation of ultraviolet light so that it shows the temporal fluctuation of activity of immobilized enzymes graphically, 3B and 3D show the results by overhead irradiations of 0.5 mW/cm² on the surface of solution, and 3C and 3E show the results by dipping irradiations of 4.4 mW/cm² at 1.9 cm from the illuminant.

In the above experiment is proven, that the longer the irradiating time is, the more effective the effect of disinfection by using ultraviolet light will become because the effect of disinfection is proportional to the product of illuminance of effective irradiation and the time of irradiation.

In regard to the effect of disinfection, the number of active microbes of immobilized enzymes at first were at the order of 10⁶ per 1 g of immobilized enzymes, and after two hours of overhead irradiation decreased to the order of 10². On the other hand dipping irradiation resulted in even lower values i. e. at one order in the number of active microbes of immobilized enzymes compared to use of overhead irradiation.

In regard to the activity of immobilized enzymes, there was no decrease of activity by using overhead irradiaton. By using dipping irradiation the activity lowered after one hour of irradiation, and then it declined to almost half after three hours from the beginning of irradiation.

### EXAMPLE 2

Supposing that the reason of the lowering of activity of immobilized enzymes by dipping irradiation mentioned above is due to the illuminating power of ultraviolet light, another result was achieved as shown in Fig. 4. Here the experiment of overhead irradiation to soluble enzymes solution was made by varying the illuminating power of ultraviolet light. In Fig. 4, the horizontal axis indicates the time of irradiation of ultraviolet light, and the vertical axis indicates the exponent of activity of enzymes. In addition the power thereof were varied in four types as follows: 4A, "control", with no irradiation of ultraviolet light, at 0.5 mW/cm² as 4B, 1.3 mW/cm² as 4C and 3.0 mW/cm² as 4D.

In the above experiment is proven, that the stronger the illumination power is, the greater the lowering of activity. At an illuminating power of more than 0.5 mW/cm² of ultraviolet light, the activity declined to almost half after two hours of irradiation. In this way it is proven, that limit of illuminating power of ultraviolet light with minimum lowering of enzymes is 0.5 W/cm².

### EXAMPLES 3

A comparison was made between the effect of disinfection by using overhead irradiation disclosed in Example 2 and dipping irradiation of ultraviolet light at 0.5 mW/cm², and the disinfection by using sodium hypochlorite. Though sodium hypochlorite is inappropriate to disinfection of immobilized biocatalyst because sodium hypochlorite disactivates immobilized biocatalyst. However it is stonger in disinfecting power than the conventional surface active agent like quaternary ammonium salt, 10 - 50 % of propylene glycol or the like, so it was chosen for this experiment. In addition, in this experiment protease "S" was used as immobilized enzyme, and #8, #9, #10 and #11 of "chitopearls" as carriers. Said carriers were taken after being contaminated by planting heat-resisting germs of Bacillus subtilis NCOD 2130 in acid caseine solution.

First 40 ml of a sodium hypochlorite solution with an available chlorine concentration of 20 ppm was prepared to which extent the activity of enzymes would not be affected. 5 g of the said immobilized enzymes are added, and then the said immobilized enzymes are stirred for ten minutes. Results are in the following TABLE.

| Carrier | Number of microbes before disinfection | Number of microbes after disinfection |
|---|---|---|
| #8 | 8.2 · 10⁴ | 4.5 · 10⁴ |
| #9 | 4.0 · 10³ | 1.2 · 10⁴ |
| #10 | 7.0 · 10⁴ | 5.1 · 10⁴ |
| #11 | 1.2 · 10⁵ | 1.0 · 10⁴ |

This experiment resulted in a disinfection by sodium hypochlorite. The kind of carrier did not made any particular difference in disinfection.

A result of an experiment by using overhead irradiation and dipping irradiation of ultraviolet light at the illuminating power of 0.5 mW/cm² is shown as Fig. 5.

Dotted lines in Fig. 5 indicate over the time of irradiation of ultraviolet light (horizontal axis) the logarithmic value of the number of active microbes of immobilized enzymes on the vertical axis. Solid lines indicate over the time of irradiation of ultraviolet light (horizontal axis) the exponent of activity of immobilized enzymes for the vertical axis. And indications #8, #9, #10 and #11 are the numberings of kind of "chitopearl" used as carriers.

In this experiment both overhead irradiation and dipping irradiation of ultraviolet light resulted in disinfection when whatever carriers were used. After 30 minutes of irradiation the logarithmic value of the number of active microbes of immobilized enzymes decreased at 1 - 2 orders. Especially, "chitopearl" #9 gave a particular effect. The number of microbes were at zero after an hour of irradiation. Therefore it is proven that the smoother the construction of surface of the carrier is, the more effective the disinfection will be.

Comparing to this effects of disinfection, the exponent of activity of immobilized enzymes were not changed to that extent when any of "chitopearls" #8, #9, #10 and 11 as carriers were used. The activity of immobilized enzymes were not lost.

As above stated, the disinfection of immobilized biocatalyst by using overhead irradiation and dipping irradiation of ultraviolet light beyond the upper limit of illuminating power of 0.5 mW/cm² gives particular effect both in disinfection and in keeping acticity of enzymes.

The apparatus according to the present invention will be explained with reference to the drawings hereinafter:

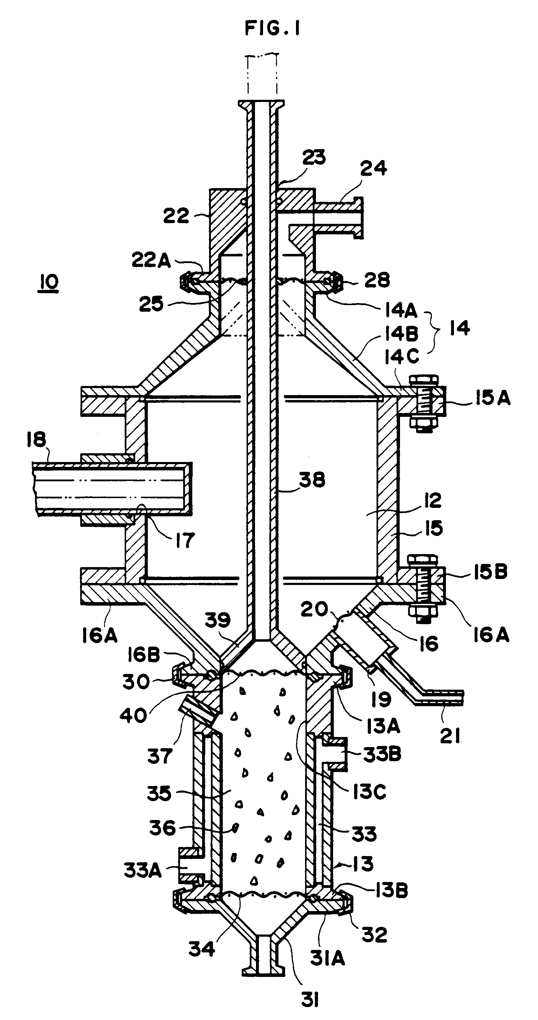

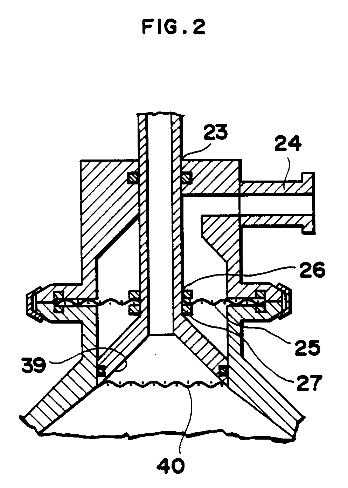

Fig. 1 and Fig. 2 show an example of an embodiment. Reference numeral 10 shows a reactor wherein a hollow column chamber 13 is connected with a down portion of a hollow fluidized chamber 12. The fluidized chamber 12 is formed by a central cylinder portion 15, an upper funnel portion 14 and lower funnel portion 16. The lower flange 14C of the upper funnel portion 14 and the upper flange 15A of the cylinder portion 15, and the lower flange 15B of the cylinder portion 15 und upper flange 16A of the lower funnel portion 16 are bolted together, respectively.

The washing gateway 19 is penetrated at the down portion of the lower funnel portion 16. Said is provided with a filter 20 at the surface level of the wall of the lower funnel portion 16. The other end of the washing gateway 19 is connected with a tube 21. Further, the washing gateway 19 is usually used for the exit of washing liquid but the use as an entrance is also possible.

The upper funnel portion 14 is constructed as the connection from the upper level, with the upper flange 14A, funnel 14B and the lower flange 14C, respectively. At the upper flange 14A of the upper funnel portion 14, the lower flange 22A of the upper cylinder portion 22 is connected by a clamp coupling 28. In the upper cylinder portion 22 there is provided a penetrable hole 23 for the inner tube 38 at the top surface, and a washing gateway 24 at the top end. Between the upper flange 14A and the lower flange 22A of the upper cylinder portion 22 engages an outflow-preventing net 25 At the center of the said net there is provided an engaging hole 26 and O-ring 27 engageable with the inner tube 38. Further, the washing gateway 24 is usually used for the exit of washing liquid, but the use as an entrance is also possible.

At the lower portion of the lower funnel portion 16 there is provided a flange 16B which is connected with the upper flange 13A of the column chamber 13 by a clamp coupling 30. The lower flange 13B of the column chamber 13 is connected with the upper flange 31A of the funnel tube 31 by a clamp coupling 32. Around the perimeter of the column chamber 13 there is provided a jacket 33. 33A and 33B are an entrance and an exit, respectively, for the cooling water, to be filled up in the jacket 33.

Reference numeral 38 shows an inner tube penetrating through the hole 23 of the upper cylinder portion 22 and the engaging hole 26 of the outflow-preventing net 25. At the top end of the inner tube 38 a funnel head opening 39 is extending. The head opening 39 is bridged by an outflow-preventing net 40.

The end of the perimeter of the head opening 39 is connected to an inner wall 13C of the column chamber 13. The inner tube 38 is available for flushing. When the washing and disinfecting of immobilized biocatalyst are desired, they are possible when the inner tube 38 is raised, and the outside of the head opening 39 is connected with the upper inside of the upper flange 14A of the upper funnel portion 14.

At the bottom of the column chamber 13 there is provided an outflow-preventing net 34. Immobilized biocatalyst 36 is filled up in the filling chamber 35 of the column chamber 13, secluded by an outflow-preventing net 40, an inner wall 13C and a lower outflow-preventing net 34. Reference numeral 37 is a pick-up gate for samples of immobilized biocatalyst. Through the said pick-up gate immobilized biocatalyst is picked up when necessary e.g. for investigating the condition of the washing, the disinfecting and the potential of elution of the said immobilized biocatalyst.

On the other hand, at the trunk of the central cylinder portion 15 of the fluidized chamber 12, there is an opening 17 for an ultraviolet lamp 18. Said lamp is insertable into the inside of the central cylinder portion 15. Through the inner tube 38 is connected with the inner tube 13C of the column chamber 13, the use of ultraviolet lamp is possible when said ultraviolet lamp is constructed so as not to prevent the movement of the inner tube 38 in the fluidized chamber.

The Elution, washing and disinfection of immobilized biocatalyst within the reactor provided with fluidized chamber will be explained according to Fig. 1. The solution with matrix is declined from the solution gate of the inner tube 38 and then flows to the column chamber 35 trough the outflow-preventing net 40. Then it reacted by immobilized biocatalyst. In this case the head opening 39 is connected with the inner wall 13C of the column chamber 13. The reacted solution passes through the lower outflow-preventing net 34 and is transferrred to the next process.

During this reaction the washing of the inside of the fluidized chamber 12 is possible. When the washing liquid is introduced at high pressure from the washing gateway 24, the washing of the inside of the fluidized chamber is possible separately from the washing of the immobilized biocatalyst. The washing of the inside of the fluidized chamber 12 is possible when the washing liquid is introduced from the washing gateway 24 and exhausted from the gateway 19.

When the washing and the disinfecting of the immobilized biocatalyst is desired because loss of pressure by adhesion of protein or the like occurs, the inner tube 38 is raised up and the top end of the outside of the head opening 39 is connected with the bottom of the engaging hole 26 of the upper outflow-preventing net 25.

Then the washing liquid is introduced through the funnel tube 31. In this process immobilized biocatalyst is floated inside the fluidized chamber 12. Before the immobilized biocatalyst is submerged into the column chamber 13, the inner tube 38 is dropped and connected with the inner wall 13C of the column chamber 13.

Through said operation in the fluidized chamber 12, there occurs the scroll in the fluidized chamber 12 when the washing liquid is introduced from the washing gateway 24. The immobilized biocatalyst is stirred and washed by said scroll of the washing liquid. Then the washing liquid is exhausted through the washing gateway 19. In this case, different from the prior art, washing without disactivating immobilized biocatalsysts is possible because washing of only the immobilized biocatalyst in the fluidized chamber 12 is possible. After the said washing, the ultraviolet lamp 18 is inserted into the ultraviolet opening 17 and into the inside of the central cylinder portion 15. Then the disinfection of the immobilized biocatalyst is performed.

While the washing and the disinfecting of immobilized biocatalyst is operated in the fluidized chamber 12, in the inside of the column chamber 13 the washing is possible separately by introducing the washing liquid through funnel tube 31 and by exhausting to the top of the inner tube from the funnel head opening 39 to the inner tube 38.

After washing the inside of the column chamber 13, the washing liquid is exhausted. Then the inner tube 38 is raised again and the top end of the outside of the head opening 39 is connected with the bottom of the engaging hole 26 of the upper outflow-preventing net 25. Until this step the washing and the disinfecting is completed and the immobilized biocatalyst floating in the fluidized chamber 12 is submerged into the column chamber 13. Then the end of the perimeter of the head opening 39 of the inner tube 38 is connected with the inner wall 13C of the column chamber 13. Then the remained liquid in the fluidized chamber 12 is exhausted outside from the washing gateway 19 through the filter 20.

## Claims

1. A reactor (10) for washing and disinfecting immobilized biocatalysts, comprising:
- a fluidized chamber (12) with an opening at its lower position (16),
- a column chamber (13) connected to said fluidized chamber (12),
- an inner tube (38) extending through the fluidized chamber (12) to the column chamber, said tube (38) being movable vertically inside said fluidized chamber (12), whereby in the elevated position of the tube (38) the fluidized chamber and the column chamber are connected,
- a lower opening of said inner tube contacting the column chamber (13) in the lower position of the tube (38), thereby separating the column chamber (13) and the fluidized chamber (12), but connecting the tube (38) and the column chamber (13),
characterized in
that the fluidized chamber (12) is provided with an ultraviolet lamp (18) insertable into the inside of said fluidized chamber (12).

2. A reactor according to claim 1, characterized in that the ultraviolet lamp (18) provide an illuminating power of less than 0.5 mW/cm}.

3. A reactor according to claim 1 or 2, characterized in that the ultraviolet lamp (18) is insertable through an opening (17) in a central portion (15) of the fluidized chamber (12).

4. A reactor according to at least one of the preceding claims, characterized in that a washing gateway (19), provided with a retaining net (20), is connected to the fluidized chamber (12).

## Patentansprüche

1. Reaktor (10) zur Reinigung und Entkeimung immobilisierter Biokatalysatoren, umfassend:
- einen Fließraum (12) mit einer Öffnung an seinem unteren Ende (16),
- einen mit dem genannten Fließraum (12) in Verbindung stehenden Säulenraum (13),
- ein durch den Fließraum (12) zum Säulenraum hin verlaufendes Innenrohr (38), welches in dem genannten Fließraum (12) senkrecht beweglich ist, wobei Fließraum und Säulenraum in Verbindung stehen, wenn sich das Rohr (38) in seiner oberen Position befindet,
- eine untere Öffnung des genannten Innenrohrs, die in Kontakt mit dem Säulenraum (13) steht, wenn sich das Rohr (38) in seiner unteren Position befindet, wodurch der Säulenraum (13) und der Fließraum (12) getrennt werden, während das Rohr (38) und der Säulenraum (13) verbunden werden,
dadurch gekennzeichnet,
daß der Fließraum (12) mit einer Ultraviolett-Lampe (18) ausgestattet ist, die in das Innere des genannten Fließraums (12) einführbar ist.

2. Reaktor nach Anspruch 1, dadurch gekennzeichnet, daS die Ultraviolett-Lampe (18) eine Leuchtkraft von höchstens 0,5 mW/cm2 aufweist.

3. Reaktor nach Anspruch 1 oder 2, dadurch gekennzeichnet, daß die Ultraviolett-Lampe durch eine Öffnung (17) in einem Mittelteil (15) des Fließraums (12) einführbar ist.

4. Reaktor nach mindestens einem der vorangegangenen Ansprüche, dadurch gekennzeichnet, daß eine mit einem Rückhaltenetz (20) ausgestattete Reinigungsleitung (19) mit dem Fließraum (12) in Verbindung steht.

## Revendications

1. Réacteur (10) pour laver et désinfecter des biocatalyseursimmobilisés, comprenant :
- une chambre fluidisée (12) avec une ouverture à sa partie inférieure (16),
- une chambre colonne (13) reliée à cette chambre fluidisée (12),
- un tube interne (38) s'étendant à travers la chambre fluidisée (12) vers la chambre colonne, ce tube (38) pouvant se déplacer verticalement à l'intérieur de cette chambre fluidisée (12), de sorte que dans la position haute du tube (38) la chambre fluidisée et la chambre colonne sont reliées ensemble,
- l'orifice inférieur de ce tube interne venant au contact de la chambre colonne (13) dans la position basse du tube (38), séparant ainsi la chambre colonne (13) et la chambre fluidisée (12), mais reliant le tube et la chambre colonne (13),
caractérisé en ce que la chambre fluidisée (12) est munie d'une lampe ultraviolette (18) pouvant être insérée à l'intérieur de cette chambre fluidisée (12).

2. Réacteur selon la revendication 1, caractérisé en ce que la lampe ultraviolette (18) possède un pouvoir éclairant inférieur à 0,5 mW/cm.

3. Réacteur selon les revendications 1 ou 2, caractérisé en ce que la lampe ultraviolette (18) peut être insérée à travers une ouverture (17) dans la position centrale (15) de la chambre fluidisée (12).

4. Réacteur selon au moins une des revendications précédentes, caractérisé en ce qu'un orifice de lavage (19), muni d'un filtre de toile métallique (20), est relié à la chambre fluidisée (12).
